# EUROPÄISCHE PATENTSCHRIFT

(11) **EP 2 853 188 B1**
(45) Veröffentlichungstag und Bekanntmachung des Hinweises auf die Patenterteilung: **20.04.2016**
(21) Anmeldenummer: 14186105.4
(22) Anmeldetag: 24.09.2014
(51) Int. Cl.: A61B 1/00, A61B 1/12, A61B 90/00, G02B 23/24, A61B 1/06, A61B 1/002, A61B 5/00

(54) **Kühlung eines medizinischen Instruments**
Cooling of a medical instrument
Refroidissement d'un instrument médical

(30) Priorität: 24.09.2013 DE 102013110587
(43) Veröffentlichungstag der Anmeldung: 01.04.2015
(73) Patentinhaber: Karl Storz GmbH & Co. KG, 78532 Tuttlingen (DE)
(72) Erfinder: Heni, Andreas, 78567 Fridingen (DE); Kupferschmid, Markus, 78576 Emmingen-Liptingen (DE); Czupalla, Christian, 78224 Singen (DE)
(74) Vertreter: Wimmer, Stephan

(56) Entgegenhaltungen:
- WO-A1-2011/058505
- US-A- 3 299 884
- US-A1- 2003 214 579
- US-A1- 2005 158 687
- US-A1- 2006 004 276

## Beschreibung

Die vorliegende Erfindung ist auf ein Exoskop, ein Endoskop oder ein anderes medizinisches Instrument und insbesondere auf die Kühlung des medizinischen Instruments bezogen.

Exoskope, Endoskope und andere medizinische Instrumente können Bildsensoren, Prozessoren und andere Schaltungen zur Verarbeitung von Bild- und anderen Daten, Leistungselektronik, Leuchtdioden oder andere Lichtquellen und andere Wärmequellen enthalten. Herkömmlich wird die von diesen Wärmequellen erzeugte Wärmeleistung vor allem abgeführt durch freie bzw. natürliche Konvektion, die durch die Erwärmung der das medizinische Instrument umgebenden Luft angetrieben wird. Die fortschreitende Miniaturisierung elektronischer Bauteile, ihre zunehmende Leistungsfähigkeit und ihre Verfügbarkeit zu immer geringeren Kosten führen dazu, dass medizinische Instrumente eine steigende Anzahl elektronischer Bauteile mit einer steigenden Wärmeleistung enthalten. Die Wärmeabfuhr durch freie Konvektion reicht deshalb nicht mehr in allen Fällen aus. Ein herkömmlicher Lüfter bzw. Verdichter zur Erzwingung von Konvektion bzw. eines kühlenden Fluidstroms kann nicht ohne Weiteres gereinigt und autoklaviert werden und kommt deshalb zur Verwendung an vielen medizinischen Instrumenten nicht in Betracht.

Eine Aufgabe der vorliegenden Erfindung besteht darin, ein verbessertes medizinisches Instrument zu schaffen, das insbesondere besser gekühlt ist und gleichzeitig einfach gereinigt und vorzugsweise autoklaviert werden kann.

In US 2003/0214579 A1 sind mit eigener Kraft angetriebene verschluckbare Kapseln beschrieben. Die Kapseln weisen je eine Beleuchtungseinheit und ein Bilderzeugungssystem mit einer Kamera auf. Jede Kapsel weist ferner Wände auf, die ein Volumen umschließen, in dem unter anderem bildgebende und steuernde Komponenten angeordnet sind. Jede Kapsel weist ferner einen Kanal auf, in dem ein magnetischer Rotor mit mehreren Rotorblättern angeordnet ist. Jede Kapsel weist eine Statoreinheit mit elektrisch leitfähigen Spulen im Volumen auf, um den Rotor anzutreiben.

In US 2006/0004276 A1 ist eine In-Vivo-Vorrichtung beschrieben. Die Vorrichtung weist in einem Gehäuse eine Lichtquelle und eine bildgebende Vorrichtung mit einem bildgebenden Sensor auf. Ferner weist die Vorrichtung einen magnetischen Propeller auf, der mittels mehrerer von Wechselströmen durchflossenen Spulen in der Art eines bürstenlosen Motors angetrieben wird. Kanäle können Flüssigkeiten zum Propeller leiten.

Diese Aufgabe wird durch die Gegenstände der unabhängigen Ansprüche gelöst.

Weiterbildungen sind in den abhängigen Ansprüchen angegeben.

Ein medizinisches Instrument umfasst eine flüssigkeitsdichte Hülle, eine Wärmequelle, einen Oberflächenbereich, der mit der Wärmequelle thermisch gekoppelt ist, ein Laufrad außerhalb der hermetisch dichten Hülle zum Erzeugen eines Fluidstroms an der Oberfläche, einen Magneten an dem Laufrad und eine Einrichtung zum Erzeugen eines veränderlichen Magnetfelds zum Bewegen des Laufrads.

Ein medizinisches Instrument umfasst eine fluiddichte oder zumindest flüssigkeitsdichte Hülle, eine Wärmequelle, einen Oberflächenbereich, der mit der Wärmequelle thermisch gekoppelt ist, ein Laufrad außerhalb der Hülle zum Erzeugen eines Fluidstroms an der Oberfläche, einen Magneten an dem Laufrad und eine Einrichtung zum Erzeugen eines veränderlichen Magnetfelds zum Bewegen des Laufrads, wobei die Einrichtung innerhalb der fluiddichten Hülle angeordnet ist.

Das medizinische Instrument ist insbesondere ein Exoskop oder ein Endoskop.

Ein Exoskop ist eine zum extrakorporalen Gebrauch vorgesehene und ausgebildete Vorrichtung zur visuellen Inspektion bzw. Betrachtung von Objekten in der Medizin, insbesondere von Objekten an oder nahe äußeren Oberflächen eines menschlichen oder tierischen Körpers. Im Unterschied zu einem Endoskop ist ein Exoskop nicht dazu ausgebildet, um durch eine kleine natürliche oder künstliche Öffnung in einen natürlichen oder künstlichen Hohlraum eingeführt zu werden. Ein Exoskop ist vielmehr für eine Betrachtung eines Objekts ausgebildet, das zumindest während der Betrachtung, insbesondere während einer Operation, von außen sichtbar ist. Entsprechend befindet sich das Exoskop während seines bestimmungsgemäßen Einsatzes vollständig außerhalb des menschlichen oder tierischen Körpers und weist im Unterschied zum Endoskop nicht unbedingt einen langen dünnen Schaft auf.

Ein Exoskop kann eine oder mehrere Kameras oder lichtempfindliche Bildsensoren zur zweidimensionalen oder dreidimensionalen Erfassung und Darstellung, beispielsweise auf einem Bildschirm, aufweisen. Alternativ ist ein Exoskop monokular oder binokular für die unmittelbare Betrachtung mit dem menschlichen Auge ausgebildet. Ein Exoskop ist in der Regel für eine Gegenstandsweite im Bereich von einigen oder wenigen Zentimetern oder wenigen Dezimetern ausgebildet oder optimiert. Ein Exoskop kann eine starke Vergrößerung aufweisen, die eine Auflösung ermöglicht, die mit bloßem Auge nicht erreichbar ist, und damit Eigenschaften einer Lupe bzw. Stereolupe oder eines Mikroskops bzw. Stereomikroskops aufweisen. Vom Mikroskop bzw. Stereomikroskop unterscheidet sich das Exoskop in der Regel durch eine größere Gegenstandsweite.

Die Hülle ist insbesondere hermetisch dicht. Die hermetisch dichte Hülle umschließt insbesondere den Beobachtungsstrahlengang. Ferner kann die hermetisch dichte Hülle den Beleuchtungsstrahlengang und/oder andere Teile des medizinischen Instruments umschließen. Alle innerhalb der hermetisch dichten Hülle angeordneten Komponenten und Bauteile des medizinischen Instruments sind dort insbesondere vor Wasserdampf und anderen schädlichen Fluiden geschützt. Alle innerhalb der hermetisch dichten Hülle angeordneten Komponenten und Bauteile des medizinischen Instruments können nicht verschmutzen und müssen entsprechend auch nicht gereinigt werden. Die äußere Oberfläche der hermetisch dichten Hülle ist insbesondere möglichst weitgehend glatt und konvex ausgeführt, um eine Reinigung zu vereinfachen.

Die Wärmequelle umfasst insbesondere einen Bildsensor, einen Prozessor oder einen anderen Schaltkreis zur Aufbereitung oder Verarbeitung von Bilddaten und/oder anderen Daten, eine Leuchtdiode oder ein anderes Leuchtmittel und/oder Leistungselektronik zur Versorgung eines Leuchtmittels, eines Prozessors oder eines Schaltkreises mit elektrischer Leistung. Die Wärmequelle ist insbesondere in einem Handgriff oder einer Handhabe am proximalen Ende des medizinischen Instruments und/oder am distalen Ende des medizinischen Instruments angeordnet. Die Wärmequelle ist insbesondere innerhalb der hermetisch dichten Hülle angeordnet. Das Exoskop kann mehrere der beschriebenen Wärmequellen aufweisen.

Der mit der Wärmequelle thermisch gekoppelte Oberflächenbereich ist insbesondere ein Bereich der äußeren Oberfläche der hermetisch dichten Hülle. Der Oberflächenbereich ist mit der oder den Wärmequellen des medizinischen Instruments insbesondere durch Wärmeleitung, Strahlung und/oder Konvektion innerhalb der hermetisch dichten Hülle gekoppelt.

Das Laufrad ist insbesondere zum Erzeugen eines Umgebungsluft-Stroms oder eines Stroms von Kohlenstoffdioxid, Wasser oder einem anderen Umgebungsmedium des medizinischen Instruments ausgebildet. Das Laufrad kann einen Fluidstrom an der mit der Wärmequelle thermisch gekoppelten Oberfläche durch Fördern von Umgebungsluft zu dem Oberflächenbereich hin und/oder durch Fördern von Umgebungsluft von dem Oberflächenbereich weg erzeugen. Das Laufrad umfasst insbesondere mehrere Dauermagneten mit alternierend orientierter Polung.

Die Einrichtung zum Erzeugen eines veränderlichen Magnetfelds ist insbesondere zum Erzeugen eines rotierenden oder im Wesentlichen rotierenden Magnetfelds ausgebildet. Die Einrichtung umfasst insbesondere mehrere starr angeordnete Elektromagneten bzw. Spulen. Die Einrichtung zum Erzeugen eines veränderlichen Magnetfelds und das Laufrad wirken insbesondere wie die Stator und Rotor eines Synchron- oder Asynchron-Drehstrommotors zusammen.

Eine Einrichtung zur Leistungsversorgung der Elektromagneten und insbesondere zum Erzeugen von Strömen mit unterschiedlichen Phasen in den Elektromagneten kann insbesondere innerhalb der hermetisch dichten Hülle vorgesehen sein. Alternativ kann Wechselströme für die Elektromagneten durch eine separate Vorrichtung bereitgestellt und mittels elektrischer Leitungen zu dem medizinischen Instrument übertragen werden.

Alternativ umfasst die Einrichtung zum Erzeugen eines veränderlichen Magnetfelds einen oder mehrere Dauer- bzw. Permanentmagneten, die um eine Achse rotierbar und mit einem Elektromotor, einem Ultraschallmotor oder einem anderen Antrieb gekoppelt sind.

Das Laufrad kann mit einer glatten Oberfläche versehen sein kann, die ohne Weiteres gereinigt werden kann, oder als Einwegprodukt ausgebildet sein, das nach einer Verwendung entsorgt und durch ein neues und steriles Laufrad ersetzt wird. Die Einrichtung zum Erzeugen eines veränderlichen Magnetfelds ist innerhalb der hermetisch dichten Hülle vor Verschmutzung und vor der Einwirkung von Wasserdampf und anderen schädlichen Fluiden geschützt. So kann die Anordnung lediglich des Laufrads außerhalb der Hülle, jedoch der Einrichtung zum Erzeugen eines veränderlichen Magnetfelds innerhalb der hermetisch dichten Hülle die Reinigung des medizinischen Instruments vereinfachen oder überhaupt erst ermöglichen.

Das Laufrad ermöglicht durch die Erzeugung eines Fluidstroms an der mit dem Wärmequelle thermisch gekoppelten Oberflächenbereich eine wirksame Abfuhr der von der Wärmequelle erzeugten thermischen Leistung. Das medizinische Instrument kann deshalb beispielsweise mehr und komplexere Schaltungen zur Aufbereitung und Verarbeitung von Bilddaten, eine stärkere Lichtquelle bzw. ein helleres Leuchtmittel und/oder andere Abwärme erzeugende Funktionen aufweisen.

Bei einem medizinischen Instrument, wie es hier beschrieben ist, ist die Einrichtung zum Erzeugen eines veränderlichen Magnetfelds insbesondere zum Erzeugen eines rotierenden Magnetfelds vorgesehen und ausgebildet.

Die Einrichtung zum Erzeugen eines veränderlichen Magnetfelds umfasst insbesondere mindestens drei Elektromagneten, die dafür vorgesehen sind, dass in ihnen Wechselströme mit einer gegenseitigen Phasendifferenz von 120 Grad fließen.

Bei einem medizinischen Instrument, wie es hier beschrieben ist, ist die Einrichtung zum Erzeugen eines veränderlichen Magnetfelds insbesondere ferner zur magnetischen Lagerung des Laufrads und ausgebildet.

Durch eine magnetische und damit berührungslose Lagerung des Laufrads können Verschleiß und Abrieb vermieden, die Reibung reduziert oder ganz vermieden und damit die erforderliche Antriebsleistung reduziert und ein geräuscharmer Betrieb ermöglicht werden. Um im Fall einer nicht vorgesehenen äußeren Einwirkung oder eines Versagens der Einrichtung zum Erzeugen eines veränderlichen Magnetfelds eine Kollision zwischen dem Laufrad und anderen Teilen des medizinischen Instruments, Abrasion, Beschädigung oder Zerstörung des Laufrads und eine Kontamination eines Patienten mit Abriebpartikeln zu vermeiden, kann zusätzlich ein Notlauf-Lager vorgesehen sein. Das Notlauf-Lager umfasst beispielsweise ein Bauteil in Gestalt einer Lagerschale aus Polytetrafluorethylen (PTFE; auch bekannt unter dem Markennamen Teflon) oder einem anderen zähen Material, das eine geringe Reibung ermöglicht.

Bei einem medizinischen Instrument, wie es hier beschrieben ist, ist das Laufrad insbesondere zum Erzeugen eines Fluidstroms in Richtung von proximal nach distal vorgesehen und ausgebildet.

Ein medizinisches Instrument, wie es hier beschrieben ist, ist insbesondere ausgebildet, um einen Fluidstrom am distalen Ende des medizinischen Instruments zu erzeugen.

Dazu können das Laufrad und optional eine oder mehrere Leiteinrichtungen zum Leiten eines vom Laufrad erzeugten Fluidstroms an oder nahe dem proximalen Ende des medizinischen Instruments angeordnet und ausgebildet sein, um einen Fluidstrom von proximal nach distal zu erzeugen.

Bei einem medizinischen Instrument, wie es hier beschrieben ist, ist das Laufrad insbesondere an oder nahe dem distalen Ende des medizinischen Instruments angeordnet.

Bei einem medizinischen Instrument, wie es hier beschrieben ist, ist das Laufrad insbesondere zum Erzeugen eines Fluidstroms in Richtung von distal nach proximal vorgesehen und ausgebildet. Dazu ist das Laufrad insbesondere an oder nahe dem distalen Ende des medizinischen Instruments angeordnet.

Bei einem medizinischen Instrument, wie es hier beschrieben ist, ist das Laufrad insbesondere in einer Richtung parallel zu einer Längsachse des medizinischen Instruments verschiebbar.

Die Verschiebbarkeit des Laufrads in einer Richtung parallel zur Längsachse des medizinischen Instruments kann eine vollständige Reinigung und Sterilisierung des medizinischen Instruments vereinfachen oder erst ermöglichen. Das Laufrad ist insbesondere zwischen zwei oder mehr Positionen, in denen es jeweils magnetisch gehalten und antreibbar ist, verschiebbar. Das Laufrad kann zwischen diesen Positionen manuell, magnetisch oder auf andere Weise angetrieben verschiebbar sein.

Bei einem medizinischen Instrument, wie es hier beschrieben ist, kann das Laufrad insbesondere von anderen Teilen des medizinischen Instruments zerstörungsfrei und reversibel getrennt werden.

Insbesondere kann das Laufrad von anderen Teilen bzw. vom Rest des medizinischen Instruments durch eine Verschiebung nach distal bis über das distale Ende des medizinischen Instruments hinaus getrennt werden. Alternativ kann das Laufrad durch eine Verschiebung nach proximal bis über das proximale Ende des medizinischen Instruments hinaus separiert werden. Die Abnehmbarkeit des Laufrads kann die Reinigung des medizinischen Instruments vereinfachen und einen Austausch des Laufrads im Fall einer Beschädigung oder Zerstörung ermöglichen.

Bei einem medizinischen Instrument, wie es hier beschrieben ist, ist zumindest entweder die Einrichtung zum Erzeugen eines veränderlichen Magnetfelds zwischen mehreren Positionen verschiebbar oder es sind mehrere Einrichtungen, zum Erzeugen eines veränderlichen Magnetfelds vorgesehen, um das Laufrad an mehreren Positionen antreiben zu können.

Die insbesondere manuelle oder magnetische Verschiebbarkeit des Laufrads zwischen zumindest zwei Positionen, an denen das Laufrad jeweils rotiert werden kann, kann unterschiedliche Kühlmodi ermöglichen. Insbesondere kann das Laufrad alternativ am oder nahe dem proximalen Ende des medizinischen Instruments und an oder nahe dem distalen Ende des medizinischen Instruments angeordnet werden, um unterschiedliche Bereiche des medizinischen Instruments besonders zu kühlen. An jeder Position, an der das Laufrad gehalten und rotiert werden soll, eine Einrichtung zum Erzeugen eines veränderlichen Magnetfelds vorzusehen, kann eine starre und damit besonders robuste Ausgestaltung dieser Einrichtungen zum Erzeugen veränderlicher Magnetfelder ermöglichen. Insbesondere eine Verschiebbarkeit einer Einrichtung zum Erzeugen eines veränderlichen Magnetfelds kann eine Positionierung und einen Antrieb bzw. einen Betrieb des Laufrads an einer beliebigen oder einer nahezu beliebigen Position entlang des Pfads, entlang dessen die Einrichtung zum Erzeugen eines veränderlichen Magnetfelds verschiebbar ist, ermöglichen.

Ein medizinisches Instrument, wie es hier beschrieben ist, umfasst insbesondere ferner eine Steuerung zum Steuern der Einrichtung zum Erzeugen eines veränderlichen Magnetfelds, wobei die Steuerung ausgebildet ist, um zumindest entweder die Rotationsrichtung oder die Rotationsgeschwindigkeit des Laufrads zu steuern.

Eine Steuerung zum Steuern der Einrichtung zum Erzeugen eines veränderlichen Magnetfelds kann insbesondere durch Steuerung der Rotationsgeschwindigkeit eine Einstellung bzw. Variation der Kühlleistung und/oder durch Steuerung oder Umschaltung der Rotationsrichtung eine Verschiebung des Bereichs maximaler Kühlleitung am medizinischen Instrument ermöglichen.

Ein medizinisches Instrument, wie es hier beschrieben ist, umfasst insbesondere ferner eine Einrichtung zum Erfassen einer Temperatur der Wärmequelle, wobei die Steuerung mit der Einrichtung zum Erfassen der Temperatur gekoppelt und ausgebildet ist, um zumindest entweder die Rotationsrichtung oder die Rotationsgeschwindigkeit des Laufrads in Abhängigkeit von der Temperatur der Wärmequelle zu steuern.

Die Einrichtung zum Erfassen einer Temperatur der Wärmequelle umfasst insbesondere einen Sensor zum direkten oder indirekten Erfassen der Temperatur der Wärmequelle, der an oder in der Wärmequelle angeordnet ist. Alternativ umfasst die Einrichtung zum Erfassen der Temperatur der Wärmequelle einen Signaleingang zum Empfangen eines Sensorsignals von einem Sensor zum direkten oder indirekten Erfassen der Temperatur der Wärmequelle. Die Temperatur der Wärmequelle kann beispielsweise erfasst werden, indem die Spannung an einem Thermoelement, die Spannung, der Strom oder der Widerstand an einem Bauelement mit temperaturabhängigem Widerstand, eine Stromaufnahme der Wärmequelle, eine Versorgungsspannung an der Wärmequelle und/oder eine oder mehrere andere Parameter gemessen werden.

Ein medizinisches Instrument, wie es hier beschrieben ist, umfasst insbesondere ferner zwei Einrichtungen, zum Erfassen der Temperaturen von je einer von zwei Wärmequellen, wobei die Steuerung mit den beiden Einrichtungen zum Erfassen der Temperaturen der Wärmequellen gekoppelt und ausgebildet ist, um die Rotationsrichtung des Laufrads in Abhängigkeit von den Temperaturen der Wärmequellen zu steuern.

Insbesondere umfasst das medizinische Instrument eine erste Wärmequelle (beispielsweise Lichtquelle oder Bildsensor) am distalen Ende und eine zweite Wärmequelle (beispielsweise einen Prozessor oder eine andere Schaltung zum Verarbeiten oder Aufbereiten von Bilddaten) am proximalen Ende. Die Steuerung ist ausgebildet, um eine erste Rotationsrichtung des Laufrads und einen Fluidstrom nach distal zu steuern, wenn die erste Wärmequelle einer höheren Kühlleistung bedarf als die zweite Wärmequelle, und um eine zweite Rotationsrichtung des Laufrads und einen Fluidstrom nach proximal zu steuern, wenn die zweite Wärmequelle einer höheren Kühlleistung bedarf.

Ein medizinisches Instrument, wie es hier beschrieben ist, ist insbesondere ein Endoskop, ein Exoskop oder ein Operationsmikroskop.

Ein medizinisches Instrument, wie es hier beschrieben ist, umfasst insbesondere ferner eine Leiteinrichtung zum Leiten oder Umlenken eines von dem Laufrad erzeugten Fluidstroms.

Die Leiteinrichtung umfasst insbesondere einen Mantel zum Halten des Fluidstroms an dem mit der Wärmequelle thermisch gekoppelten Oberflächenabschnitt oder zum Lenken des Luftstroms zu dem mit der Wärmequelle thermisch gekoppelten Oberflächenabschnitt. Alternativ oder zusätzlich kann die Leiteinrichtung ein oder mehrere Leitgitter und/oder ein oder mehrere Leitschaufeln zum Leiten des Fluidstroms in eine erwünschte Richtung und/oder zum Formen eines Fluidstroms eines gewünschten Querschnitts oder eines gewünschten Geschwindigkeitsprofils umfassen.

Bei einem medizinischen Instrument, wie es hier beschrieben ist, ist insbesondere zumindest entweder das Laufrad oder die Leiteinrichtung ausgebildet, um einen zumindest abschnittsweise helikal um einen Schaft des medizinischen Instruments verlaufenden Fluidstrom zu erzeugen.

Die Leiteinrichtung umfasst dazu insbesondere helikal gekrümmte bzw. verwundene Leitschaufeln und/oder einen Mantel zum Führen eines vom Laufrad bereits helikal erzeugten Fluidstroms zu dem Schaft. Ein helikal um den Schaft verlaufender Fluidstrom kann besonders lang an dem Schaft anliegen und damit beispielsweise die Kühlwirkung des Fluidstroms vom proximal angeordneten Laufrad bis besonders weit nach distal ausdehnen.

Ein medizinisches Instrument, wie es hier beschrieben ist, umfasst insbesondere ferner eine Einrichtung zum Überführen einer laminaren Strömung in eine turbulente Strömung.

Die Einrichtung zum Überführen einer laminaren Strömung in eine turbulente Strömung umfasst insbesondere einen oder mehrere Turbulatoren oder Turbulenz- oder Vortexgeneratoren, wie sie insbesondere aus der Luftfahrt bekannt sind. Die Einrichtung zum Überführen einer laminaren Strömung in eine turbulente Strömung ist insbesondere stromaufwärts des mit der Wärmequelle thermisch gekoppelten Oberflächenabschnitts angeordnet. Durch laminare Strömung bis zu oder bis nahe dem mit der Wärmequelle thermisch gekoppelten Oberflächenabschnitt kann der Fluidstrom mit vergleichsweise geringen Verlusten bis zu dem mit der Wärmequelle thermisch gekoppelten Oberflächenabschnitt gelangen. Durch eine turbulente Strömung an dem mit der Wärmequelle thermisch gekoppelten Oberflächenabschnitt kann der Wärmeübergang zwischen der Oberfläche und dem Fluidstrom und damit die Kühlleistung verbessert werden.

### Kurzbeschreibung der Figuren

Nachfolgend werden Ausführungsformen anhand der beigefügten Figuren näher erläutert. Es zeigen:

| | |
|---|---|
| Figur 1 | eine schematische Darstellung eines Exoskops; |
| Figur 2 | eine weitere schematische Darstellung des Exoskops aus Figur 1; |
| Figur 3 | eine schematische Darstellung eines Endoskops; |
| Figur 4 | eine weitere schematische Darstellung des Endoskops aus Figur 3; |
| Figur 5 | eine schematische Darstellung eines weiteres Exoskops; |
| Figur 6 | eine schematische Darstellung eines weiteren Exoskops; |
| Figur 7 | eine weitere schematische Darstellung des Exoskops aus Fig. 6; |
| Figur 8 | eine schematische Darstellung eines weiteren Exoskops; |
| Figur 9 | eine schematische axonometrische Darstellung eines weiteren Exoskops; |
| Figur 10 | eine schematische axonometrische Schnittdarstellung des Exoskops aus Figur 9; |
| Figur 11 | eine weitere schematisch axonometrische Darstellung des Exoskops aus den Figuren 9 und 10. |

### Beschreibung der Ausführungsformen

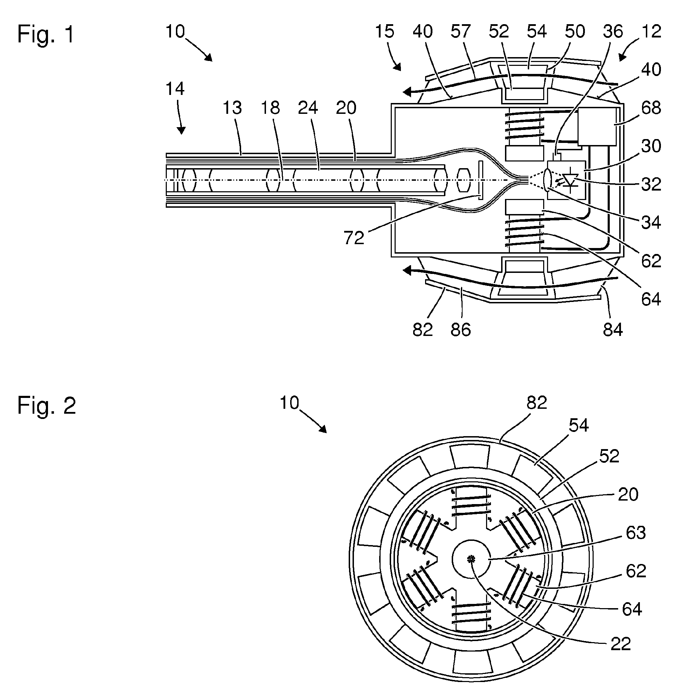

Figur 1 zeigt eine schematische Darstellung eines Exoskops 10 mit einem proximalen Ende 12 und einem distalen Ende 14. Das Exoskop 10 umfasst einen Schaft 13, der insbesondere eine kreiszylindrische oder im Wesentlichen kreiszylindrische äußere Gestalt mit einer Symmetrieachse 18 aufweist. Diese Symmetrieachse 18 wird im Folgenden auch als Längsachse des Exoskops 10 bezeichnet. Ferner umfasst das Exoskop 10 einen Handgriff 15 am proximalen Ende 12.

Die Darstellung des Exoskops 10 in Figur 1 ähnelt einer Schnittdarstellung. Abweichend von einer echten Schnittdarstellung sind einige Komponenten und Bauteile des Exoskops 10 jeweils in Draufsicht angedeutet und Schnittflächen nicht schraffiert.

Das Exoskop 10 umfasst eine hermetisch dichte Hülle 20, die insbesondere aus mehreren miteinander stoffschlüssig gefügten Teilen besteht. Innerhalb der hermetisch dichten Hülle 20 sind Lichtleitfasern 22 zum Übertragen von Beleuchtungslicht, das von einer Lichtquelle 30 am proximalen Ende 12 des Exoskops 10 erzeugt wird, zum distalen Ende 14 angeordnet. Ferner ist ein Strahlengang 24 für Beobachtungslicht innerhalb der hermetisch dichten Hülle 20 und insbesondere im Bereich des Schafts 13 vorgesehen. Der Strahlengang 24 ist zum Übertragen von Licht, das von einem zu beobachtenden Gegenstand ausgeht, zu einem im Handgriff 15 angeordneten Bildsensor 72 vorgesehen. Die Lichtquelle 30 umfasst insbesondere eine oder mehrere Leuchtdioden 32 und eine Linse 34 oder andere Einrichtungen, die eine Einkopplung eines möglichst großen Anteils des von der Leuchtdiode 32 erzeugten Lichts in die Lichtleitfasern 22 ermöglicht.

Die Lichtquelle 30, die Lichtleitfasern 22, der Bildsensor 72 und in Figur 1 nicht dargestellte Einrichtungen, zur Aufbereitung und Verarbeitung eines vom Bildsensor 72 erzeugten Bildsignals und/oder zur Steuerung der Lichtquelle 30 sind Beispiele für Wärmequellen innerhalb der hermetisch dichten Hülle 20. Da kein Stoffaustausch zwischen dem Inneren der hermetisch dichten Hülle 20 und der Umgebung des Exoskops 10 stattfindet, muss die gesamte von den Wärmequellen erzeugte Wärme über die hermetisch dichte Hülle 20 an die Umgebung des Exoskops 10 abgegeben werden.

Im Bereich des Handgriffs 15 des Exoskops 10 ist ein axialer Verdichter bzw. ein Laufrad 50 angeordnet, das insbesondere um die Längsachse 18 des Exoskops 10 rotierbar ist. Das Laufrad 50 umfasst einen Ring 52, an dessen äußerem Umfang im Wesentlichen in radialer Richtung Schaufeln 54 angeordnet sind. Proximal des Laufrads 50, insbesondere proximal der Schaufeln 54 des Laufrads 50 sind proximale Leitschaufeln 84 angeordnet. Distal des Laufrads 50 sind distale Leitschaufeln 86 angeordnet. Ein ringförmiger Mantel 82 verbindet die radial äußeren Enden der proximalen und distalen Leitschaufeln 84, 86 und umschließt das Laufrad 50. Die Schaufeln 54 des Laufrads 50 sind derart ausgebildet, dass eine Rotation des Laufrads 50 in einer vorbestimmten Rotationsrichtung um die Längsachse 18 des Exoskops 10 einen Luftstrom 57 von proximal nach distal im ringförmigen Zwischenraum zwischen der hermetisch dichten Hülle 20 und dem Mantel 82 erzeugt. Der vom rotierenden Laufrad 50 erzeugte Luftstrom 57 ist im Wesentlichen parallel zur Längsachse 18 des Exoskops 10.

Innerhalb der hermetisch dichten Hülle 20 sind ein Magnetflussleiter 62, mehrere Leiterspulen 64 am Magnetflussleiter 62 und eine mit den Leiterspulen 64 gekoppelte Steuerung 68 angeordnet. Die Steuerung 68 umfasst mehrere Leistungsquellen zum Erzeugen von Wechselströmen in den Leiterspulen 64. Der Magnetflussleiter 62, die Leiterspulen 64 und die Steuerung 68 bilden eine Einrichtung zum Erzeugen eines veränderlichen Magnetfelds. Die Steuerung 68 ist ausgebildet, um in den Magnetflussleitern 62 Wechselströme gleicher Frequenz und unterschiedlicher Phasenlage zu erzeugen, so dass die Leiterspulen 64 entsprechende magnetische Wechselfelder erzeugen. Die Steuerung 68 ist insbesondere ausgebildet, um mittels der Leiterspulen 64 ein im Wesentlichen rotierendes Magnetfeld zum Bewegen bzw. Antreiben des Laufrads 50 zu erzeugen. Damit das Laufrad 50 von dem durch die Steuerung 68 mittels der Leiterspulen 64 erzeugten rotierenden Magnetfeld angetrieben werden kann, sind der Ring 52 und/oder die Schaufeln 54 des Laufrads 50 magnetisierbar oder magnetisiert. Alternativ oder zusätzlich kann das Laufrad 50 elektrisch leitfähig ausgebildet sein, um eine Induktion von Wirbelströmen im Laufrad 50 durch ein rotierendes Magnetfeld zu ermöglichen.

Die Steuerung 68 ist mit einem oder mehreren Temperatursensoren 36 an der Lichtquelle 30 und/oder an einer oder mehreren anderen Wärmequellen innerhalb der hermetisch dichten Hülle 20 gekoppelt. Alternativ oder zusätzlich kann die Steuerung 68 einen oder mehrere Signaleingänge zum Erfassen von Strömen, Spannungen, Widerständen oder anderen Parametern, aus denen die Temperatur oder die Temperaturen von einer oder mehreren Wärmequellen berechnet oder abgeschätzt werden können, aufweisen. Die Steuerung 68 ist ausgebildet, um Ströme in den Leiterspulen 64 abhängig von der oder den Temperaturen zu steuern. Insbesondere ist die Steuerung 68 ausgebildet, um das Laufrad 50 nur bei Überschreiten einer vorbestimmten Temperaturschwelle anzutreiben, oder um das Laufrad 50 bei einem höheren Wert einer erfassten Temperatur schneller anzutreiben als bei einem niedrigeren erfassten Wert der Temperatur langsamer. Ferner kann die Steuerung 68 ausgebildet sein, um die Rotationsrichtung des Laufrads 50 umzukehren, insbesondere in Abhängigkeit von mehreren erfassten Temperaturen.

Die Steuerung 68 kann ferner ausgebildet sein, um Ströme in den Leiterspulen 64 so zu steuern, dass das Laufrad 50 weder die hermetisch dichte Hülle 20 noch den Mantel 82 noch andere Teile des Exoskops 10 berührt, sondern magnetisch gelagert berührungsfrei und damit verschleißfrei rotiert.

Die Lichtquelle 30 und ggf. weitere Wärmequellen innerhalb der hermetisch dichten Hülle 20 sind durch Wärmestrahlung, Wärmeleitung in einem das Innere der hermetisch dichten Hülle 20 ausfüllenden Medium, durch Konvektion in diesem Medium und/oder durch einen in Figur 1 nicht dargestellten Wärmeleiter mit der hermetisch dichten Hülle 20, insbesondere mit einem äußeren Oberflächenbereich 40 der hermetisch dichten Hülle 20 gekoppelt. Durch einen vom rotierenden Laufrad 50 erzeugten Luftstrom 57 entlang der mit der Wärmequelle 30, 72 gekoppelten Oberfläche 40 wird die von der Wärmequelle 30, 72 erzeugte Wärme abgeführt.

Fig. 2 zeigt eine weitere schematische Darstellung des Exoskops 10 aus Figur 1. Die Zeichenebene der Figur 2 ist orthogonal zur Zeichenebene der Figur 1 und orthogonal zur Längsachse 18 des Exoskops 10. Figur 2 zeigt das Exoskop 10 aufgeschnitten entlang einer Fläche orthogonal zur Längsachse 18 und nahe dem Magnetflussleiter 62, den Leiterspulen 64 und dem Laufrad 50. Anders als in vielen Schnittdarstellungen sind in Figur 2 ebenso wie in Figur 1 Schnittflächen nicht schraffiert dargestellt.

Der Magnetflussleiter 62 weist näherungsweise die Gestalt eines Sterns auf, dessen radiale Abschnitte von jeweils einer Leiterspule 64 umgeben sind. Der Magnetflussleiter 62 weist ferner eine zentrale Durchgangsöffnung 63 auf, in der die Lichtleitfasern 22 angeordnet sind. Die radial äußeren Stirnflächen der radialen Abschnitte des Magnetflussleiters 62 liegen an der inneren Oberfläche der hermetisch dichten Hülle 20 an. Konzentrisch zu der im dargestellten Bereich kreiszylindrischen hermetisch dichten Hülle 20 und von dieser radial beabstandet sind der Ring 52 und die Schaufeln 54 des Laufrads 50 angeordnet. Das Laufrad 50 ist vom ringförmigen Mantel 82 umgeben. Der Mantel 82 ist von den radial äußeren Enden der Schaufeln 54 des Laufrads 50 beabstandet.

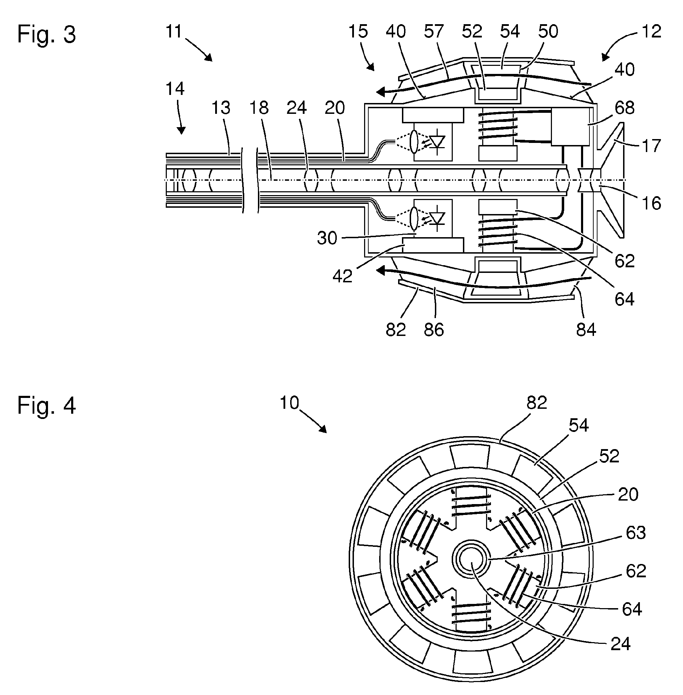

Fig. 3 zeigt eine schematische Darstellung eines Endoskops 11. Die Art der Darstellung des Endoskops 11 in Figur 3 entspricht der Art der Darstellung des Exoskops in Figur 1.

Das Endoskop 11 ähnelt in einigen Merkmalen und Eigenschaften dem anhand der Figuren 1 und 2 dargestellten Exoskop 10. Nachfolgend sind Merkmale und Eigenschaften des Endoskops 11 dargestellt, in denen sich dieses vom anhand der Figuren 1 und 2 dargestellten Exoskop unterscheidet.

Das in Figur 3 dargestellte Endoskop 11 weist einen langen Schaft 13 auf, der in Figur 3 gekürzt wiedergegeben ist. Im Schaft 13 sind Lichtleitfasern 22 zum Übertragen von Beleuchtungslicht angeordnet, die mit mehreren Lichtquellen 30 innerhalb des Handgriffs 15 am proximalen Ende 12 des Endoskops 11 gekoppelt sind. Jede einzelne Lichtquelle 30 ähnelt insbesondere der anhand der Figur 1 dargestellten Lichtquelle. Ferner ist im Schaft 13 ein Strahlengang 24 für Beobachtungslicht, das von einem zu beobachtenden Objekt ausgeht, angeordnet. Der Strahlengang 24 umfasst insbesondere eine Reihe von in Figur 3 angedeuteten Stablinsen. Der Strahlengang 24 erstreckt sich bis zu einem Okular 16 am proximalen Ende des Endoskops 11. Zur Abschirmung von Umgebungslicht und optional zur Kopplung einer Kamera mit dem Endoskop 11 ist eine Okularmuschel 17 am Okular 16 vorgesehen.

Die Lichtquellen 30 sind mittels eines Wärmeleiters 42 aus Kupfer, Aluminium oder einem anderen Material mit hoher Wärmeleitfähigkeit mit der hermetisch dichten Hülle 20 und insbesondere dem Oberflächenbereich 40 gekoppelt. Der Wärmeleiter 42 ist insbesondere kreisringförmig oder im Wesentlichen kreisringförmig ausgebildet, um die von den Lichtquellen 30 erzeugte Wärme über einen ringförmigen Oberflächenbereich 40 abzuführen und damit den vom Laufrad 50 erzeugten ringförmigen Luftstrom 57 besonders effizient zu nutzen.

Das Laufrad 50, der Mantel 82, die proximalen und distalen Leitschaufeln 84, 86, der Magnetflussleiter 62, die Leiterspulen 64 und die Steuerung 68 entsprechen oder ähneln weitgehend den oben anhand der Figuren 1 und 2 dargestellten.

Abweichend von der Darstellung in Figur 3 können ein oder mehrere Temperatursensoren an den Lichtquellen 30 und/oder an anderen Wärmequellen innerhalb der hermetisch dichten Hülle 20 mit der Steuerung 68 gekoppelt sein, ähnlich wie bei dem anhand der Figuren 1 und 2 dargestellten Exoskop.

Figur 4 zeigt eine weitere schematische Darstellung des Endoskops 11 aus Figur 3. Die Art der Darstellung in Figur 4 entspricht der Art der Darstellung in Figur 2. Insbesondere ist die Zeichenebene der Figur 4 orthogonal zur Längsachse 18 des Endoskops 11 und zur Zeichenebene der Figur 3, wobei ein Schnitt entlang einer Fläche nahe dem Laufrad 50, dem Magnetflussleiter 62 und den Leiterspulen 64 dargestellt ist. In der zentralen Durchgangsöffnung 63 des Magnetflussleiters 62 ist der Strahlengang 24 für Beobachtungslicht angeordnet.

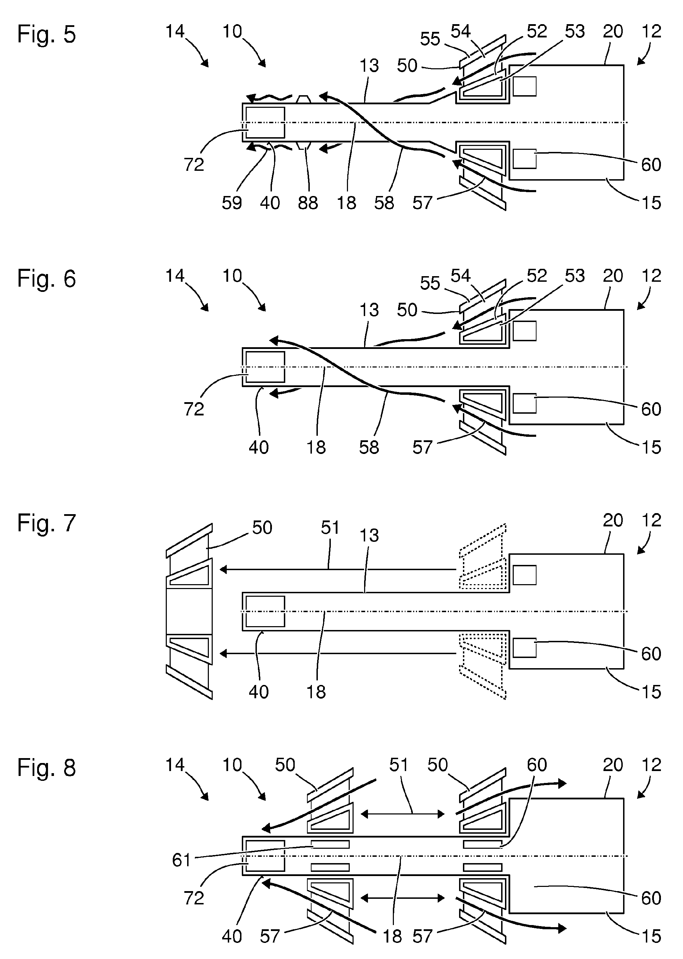

Figur 5 zeigt eine schematische Darstellung eines weiteren Exoskops 10, das in einigen Merkmalen und Eigenschaften dem anhand der Figuren 1 und 2 dargestellten Exoskop ähnelt. Ähnlich wie in den Figuren 1 bis 4 ist ein Schnitt durch das Exoskop angedeutet, wobei Schnittflächen nicht schraffiert sind. Die Zeichenebene bzw. Schnittebene der Figur 5 entspricht den Zeichen- bzw. Schnittebenen der Figuren 1 und 3. Nachfolgend sind Merkmale und Eigenschaften des Exoskops 10 dargestellt, die sich von denen des anhand der Figuren 1 und 2 dargestellten Exoskops unterscheiden.

Das Exoskop 10 weist ein proximales Ende 12, ein distales Ende 14, einen Schaft 13, der sich zum distalen Ende 14 erstreckt, und einen Handgriff 15 nahe dem proximalen Ende 12 auf. Das Exoskop 10 umfasst eine hermetisch dichte Hülle 20, innerhalb derer nahe dem distalen Ende 14 des Exoskops 10 ein Bildsensor 72 oder eine Kamera angeordnet ist. Der Bildsensor 72 stellt eine Wärmequelle dar, deren Wärme abgeführt werden muss. Ferner kann das Exoskop weitere Wärmequellen aufweisen, die in Figur 5 nicht dargestellt sind, beispielsweise innerhalb des Handgriffs 15. Zu diesen in Figur 5 nicht dargestellten Wärmequellen können Einrichtungen zur Aufbereitung oder Verarbeitung eines Bildsignals vom Bildsensor 72 sowie Leistungselektronik zur Bereitstellung elektrischer Leistung für diese Einrichtungen, für den Bildsensor 72, für eine Lichtquelle oder für andere Verbraucher sein.

Ferner ist innerhalb der hermetisch dichten Hülle 20 eine Einrichtung 60 zum Erzeugen eines veränderlichen Magnetfelds angeordnet, die insbesondere einen im Wesentlichen ringförmigen Bauraum symmetrisch zur Längsachse 18 des Exoskops 10 einnimmt. Nahe der Einrichtung 60 zum Erzeugen eines veränderlichen Magnetfelds ist außerhalb der hermetisch dichten Hülle ein Laufrad 50 angeordnet, das um die Längsachse 18 des Exoskops 10 rotieren kann. Das Laufrad 50 weist einen Ring 52 mit darin eingebetteten Magneten 53, vom Ring 52 nach radial außen ragende Schaufeln 54 und einen Ringmantel 55, der die radial äußeren Enden der Schaufeln 54 ringförmig miteinander verbindet und sie mechanisch schützt, auf. Durch Wechselwirkung eines von der Einrichtung 60 erzeugten veränderlichen, insbesondere rotierenden Magnetfelds mit den Magneten 53 im Ring 52 des Laufrads 50 wird das Laufrad ähnlich einem Rotor eines Synchron-Drehstrommotors angetrieben und rotiert um die Längsachse 18 des Exoskops 10. Die Einrichtung 60 zum Erzeugen eines veränderlichen Magnetfelds kann ferner ausgebildet sein, um das Laufrad magnetisch zu lagern bzw. berührungsfrei in einer vorbestimmen Position zu halten.

Das Laufrad 50 und insbesondere seine Schaufeln 54 sind ausgebildet, um bei Rotation um die Längsachse 18 des Exoskops 10 einen Luftstrom 57 zu erzeugen. Der vom Laufrad 50 erzeugte Luftstrom 57 setzt sich entlang des Schafts 13 in Form eines laminaren und den Schaft 13 helikal umschließenden Luftstroms 58 fort. Proximal der mit der Wärmequelle 72 thermisch gekoppelten Oberfläche 40 sind Turbulatoren 88 an der äußeren Oberfläche der hermetisch dichten Hülle 20 angeordnet, die einen Übergang von einer laminaren Strömung im Luftstrom 58 in eine turbulente Strömung im Luftstrom 59 an der Oberfläche 40 bewirken. Der turbulente Luftstrom 59 an der Oberfläche 40 verbessert den Wärmeübergang von der Oberfläche 40 zu dem Luftstrom 59 und damit die Kühlwirkung.

Das in vorbestimmter Rotationsrichtung rotierende Laufrad 50 saugt Umgebungsluft nahe der äußeren Oberfläche der hermetisch dichten Hülle 20 im Bereich des Handgriffs 15 an. Dadurch erzeugt das rotierende Laufrad 50 auch im Bereich des Handgriffs einen Luftstrom, der Wärme von in Figur 5 nicht dargestellten Wärmequellen innerhalb des Handgriffs 15 abführt.

Figur 6 zeigt eine schematische Darstellung eines weiteren Exoskops 10, das in einigen Merkmalen und Eigenschaften den anhand der Figuren 1 und 5 dargestellten Exoskopen, insbesondere dem anhand der Figur 5 dargestellten Exoskop ähnelt. Die Art der Darstellung entspricht derjenigen der Figur 5. Nachfolgend sind Merkmale und Eigenschaften des Exoskops 10 beschrieben, in denen dieses sich von dem anhand der Figur 5 dargestellten Exoskop unterscheidet.

Das in Figur 6 dargestellte Exoskop unterscheidet sich von dem anhand der Figur 5 dargestellten Exoskop insbesondere dadurch, dass keine Turbulatoren 88 vorgesehen sind. Ferner ist die hermetisch dichte Hülle 20 insbesondere im Bereich des Laufrads 50 und des Schafts 13 so ausgebildet, dass das Laufrad 50 ausgehend von der in Figur 6 dargestellten Position nach distal verschoben werden kann. Die Einrichtung 60 zum Erzeugen eines veränderlichen Magnetfelds ist zur magnetischen Lagerung des Laufrads 50, d.h. zum Halten des Laufrads 50 an der vorgesehenen und in Figur 6 dargestellten Position auch während einer Rotation ausgebildet, ähnlich wie dies oben anhand der Figuren 1 und 5 beschrieben ist.

Figur 7 zeigt eine weitere schematische Darstellung des Exoskops aus Figur 6. Die Art der Darstellung in Figur 7, insbesondere die Zeichenebene, entspricht denjenigen der Figur 6. Figur 7 zeigt das Exoskop 10 in einer weiteren Konfiguration. Das Laufrad 50 ist gegenüber der in Figur 6 gezeigten und in der Figur 7 in gestrichelten Linien angedeuteten und für die Verwendung des Exoskops 10 vorgesehenen Position in einer Richtung 51 parallel zur Längsachse 18 des Exoskops 10 und über das distale Ende 14 des Exoskops 10 hinaus verschoben. Das Laufrad 50 ist somit vom Rest des Exoskops 10 geometrisch und mechanisch getrennt und kann unabhängig gereinigt und autoklaviert sowie im Fall einer Beschädigung ohne Weiteres ausgetauscht werden.

Figur 8 zeigt eine schematische Darstellung eines weiteren Exoskops 10, das in einigen Merkmalen und Eigenschaften den anhand der Figuren 1, 2 und 5 bis 7 dargestellten Exoskopen, insbesondere dem anhand der Figuren 6 und 7 dargestellten Exoskop ähnelt. Die Art der Darstellung in Figur 8 und insbesondere die dargestellte Schnittfläche entsprechen denjenigen der Figuren 1, 3 und 5 bis 7. Nachfolgend sind Merkmale und Eigenschaften beschrieben, in denen das Exoskop 10 sich von dem anhand der Figuren 6 und 7 dargestellten Exoskop unterscheidet.

Das in Figur 8 dargestellte Exoskop umfasst eine erste Einrichtung 60 zum Erzeugen eines veränderlichen Magnetfelds nahe dem Handgriff 15 und eine zweite Einrichtung 61 zum Erzeugen eines veränderlichen Magnetfelds nahe dem distalen Ende 14 des Exoskops 10. Jede der beiden Einrichtungen, 60, 61 ist ähnlich wie die anhand der Figuren 1 bis 7 dargestellten Einrichtungen zum Erzeugen veränderlicher Magnetfelder vorgesehen und ausgebildet, um ein im Wesentlichen rotierendes Magnetfeld zu erzeugen und mittels diesem das Laufrad anzutreiben und in Rotation zu versetzen. Ferner sind insbesondere beide Einrichtungen 60, 61 zum Erzeugen veränderlicher Magnetfelder dazu vorgesehen und ausgebildet, das Laufrad 50 zu lagern bzw. während der Rotation in einer vorbestimmten Position zu halten. Das Laufrad 50 kann somit alternativ in zwei verschiedenen Positionen am Exoskops 10 betrieben werden. In Figur 8 ist das Laufrad 50 je einmal in jeder der beiden Positionen dargestellt.

Bei Betrieb des Laufrads 50 in der in Figur 8 links dargestellten distalen Position wird die Wärmequelle 72 am distalen Ende 14 des Exoskops 10 besonders intensiv gekühlt. Bei Betrieb des Laufrads 50 in der in Figur 8 rechts dargestellten proximalen Position und Umkehr der Rotationsrichtung, um einen entgegengesetzt gerichteten Luftstrom 57 zu erzeugen, wird der Handgriff 15 besonders intensiv gekühlt. Das Exoskop 10 kann eine Steuerung umfassen, die in Figur 8 nicht dargestellt ist, um durch Messungen mehrerer Temperaturen, insbesondere durch Messung der Temperaturen mehrerer Wärmequellen innerhalb der hermetisch dichten Hülle 20 zu bestimmen, welche Wärmequelle einer Kühlung am meisten bedarf. Abhängig von den gemessenen Temperaturen kann die Steuerung das Laufrad dann in unterschiedlichen Positionen und/oder mit unterschiedlichen Laufrichtungen betreiben, um besonders die Wärmequelle mit dem höchsten Kühlbedarf zu kühlen. Eine Bewegung des Laufrads 50 in Richtung 51 parallel zur Längsachse 18 des Exoskops 10 zwischen den beiden in Figur 8 dargestellten und/oder weiteren Positionen kann magnetisch oder manuell nach einer durch die Steuerung 68 erzeugten Aufforderung an einer Benutzerschnittstelle oder auf andere Weise erfolgen.

Abweichend von der Darstellung in Figur 8 können drei oder mehr Einrichtungen, zum Erzeugen veränderlicher Magnetfelder vorgesehen sein. Alternativ kann eine Einrichtung zum Erzeugen eines veränderlichen Magnetfelds innerhalb der hermetisch dichten Hülle 20 verfahrbar sein (insbesondere mittels eines Ultraschallmotors oder eines elektrischen Antriebs), um das Laufrad 50 an zwei oder mehr verschiedenen Positionen zu betreiben.

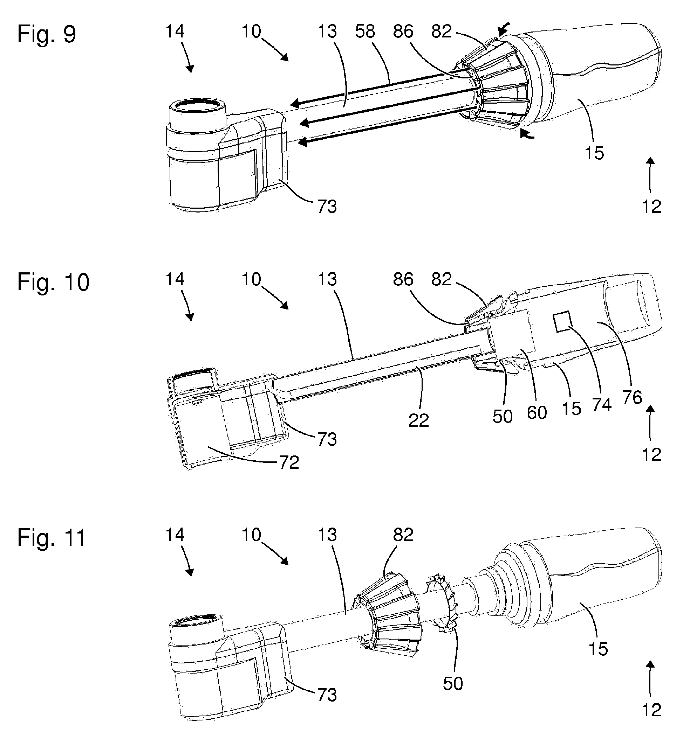

Figur 9 zeigt eine schematische axonometrische Darstellung eines weiteren Exoskops 10, das in einigen Merkmalen und Eigenschaften den oben anhand der Figuren 1, 2 und 5 bis 8 dargestellten Exoskopen ähnelt. Insbesondere weist das Exoskop Wärmequellen auf, die einer Kühlung bedürfen.

Das Exoskop 10 weist am proximalen Ende 12 einen Handgriff 15 und am distalen Ende 14 ein Kameragehäuse 73 auf. Der Handgriff 15 und das Kameragehäuse 73 sind durch einen starren und geraden Schaft 13 mit einer im Wesentlichen kreiszylindrischen Mantelfläche miteinander verbunden. Die Blickrichtung einer Kamera im Kameragehäuse 73 ist orthogonal zu der Längsachse des Schafts 13.

Im Übergangsbereich zwischen dem Handgriff 15 und dem Schaft 13 sind ein Mantel 82 und Leitschaufeln 86 in einem monolithischen oder im Wesentlichen monolithischen Bauteil angeordnet. Der Mantel 82 umgibt ein Laufrad zum Erzeugen eines dann durch den Mantel 82 und die Leitschaufeln 86 gelenkten und geformten, im Wesentlichen laminaren Luftstroms 58 entlang des Schafts 13 zum Kameragehäuse 73. Der Luftstrom 58 umspült im weiteren Verlauf das Kameragehäuse 73 und erhöht damit die Wärmeabfuhr von dessen Oberfläche.

Figur 10 zeigt eine weitere schematisch axonometrische Darstellung des Exoskops 10 aus Figur 9. Das Exoskop 10 ist in Figur 10 entlang einer Ebene aufgeschnitten dargestellt, die die Symmetrieachse der äußeren Oberfläche des Schafts 13 und die optische Achse einer Kamera 72 im Kameragehäuse 73 enthält. In dem in Figur 10 dargestellten Schnitt sind das vom Mantel 82 umgebene Laufrad 50, eine Einrichtung 60 zum Erzeugen eines veränderlichen Magnetfelds zum Antreiben des Laufrads 50 im Übergangsbereich zwischen Handgriff 15 und Schaft 13 und Bauraum 76 im Handgriff 15 für einen Prozessor oder eine andere Schaltung 74 zum Aufbereiten oder Verarbeiten eines Bildsignals oder für andere Wärmequellen erkennbar. Ferner ist ein Bündel Lichtleitfasern 22 im Schaft 13 zum Leiten von Beleuchtungslicht von einer Lichtquelle im Bauraum 76 im Handgriff 15 zum distalen Ende 14 des Exoskops 10 und eine Kamera 72 im Kameragehäuse 73 erkennbar.

Figur 11 zeigt eine weitere schematische axonometrische Darstellung des Exoskops aus den Figuren 9 und 10. Die Art der Darstellung in Figur 11 ähnelt derjenigen in Figur 9. Im Unterschied zu den Figuren 9 und 10 ist in Figur 11 das Exoskop 10 in einer Konfiguration gezeigt, die nicht zum Betrieb bzw. zur Verwendung des Exoskops 10, sondern zu dessen Reinigung geeignet ist. Der Mantel 82 mit den Leitschaufeln 86 und das Laufrad 50 sind von ihren für den Betrieb bzw. die Verwendung des Exoskops 10 vorgesehenen Positionen im Übergangsbereich zwischen Handgriff 15 und Schaft 13 ausgehend nach distal verschoben. Dadurch sind die Oberflächen des Laufrads 50 sowie Oberflächen im Bereich des Mantels und der Leitschaufeln 86 für eine Reinigung zugänglich. Die übrigen äußeren Oberflächen des Exoskops 10 sind insbesondere durch mehrere Oberflächenbauteile gebildet, die untereinander fugenfrei und insbesondere stoffschlüssig gefügt sind, um eine einfache und vollständige Reinigung zu ermöglichen.

### Bezugszeichen

- 10: Exoskop
- 11: Endoskop
- 12: proximales Ende des Exoskops 10 oder des Endoskops 11
- 13: Schaft des Exoskops 10 bzw. des Endoskops 11
- 14: distales Ende des Exoskops 10 oder des Endoskops 11
- 15: Handgriff am proximalen ende 12 des Exoskops
- 16: Okular am proximalen Ende 12 des Endoskops 10
- 17: Okularmuschel
- 18: Längsachse des Exoskops 10 bzw. des Endoskops 11

- 20: hermetisch dichte Hülle des Exoskops 10 oder des Endoskops 11
- 22: Lichtleitfasern für Beleuchtungslicht, Strahlengang für Beleuchtungslicht
- 24: Strahlengang für Beobachtungslicht

- 30: Lichtquelle innerhalb der hermetisch dichten Hülle des Exoskops 10
- 32: Leuchtdiode der Lichtquelle 30
- 34: Linse der Lichtquelle 30
- 36: Temperatursensor an der Lichtquelle 30

- 40: mit einer Wärmequelle 22, 32, 72, 74 gekoppelte Oberfläche an der hermetisch dichten Hülle 20
- 42: Wärmeleiter zwischen Wärmequelle 30 und Oberfläche 40

- 50: Laufrad bzw. axialer Verdichter
- 51: Richtung, in der das Laufrad 50 vom Rest des Exoskops 10 bzw. des Endoskops 11 getrennt werden kann
- 52: Ring des Laufrads 50
- 53: Magnet im Ring 52
- 54: Schaufel des Laufrads 50
- 55: Ringmantel am Laufrad 50
- 57: vom rotierenden Laufrad 50 erzeugter Luftstrom
- 58: laminarer Luftstrom
- 59: turbulenter Luftstrom

- 60: Einrichtung zum Erzeugen eines veränderlichen Magnetfelds
- 61: weitere Einrichtung zum Erzeugen eines veränderlichen Magnetfelds
- 62: Magnetflussleiter
- 63: zentrale Durchgangsöffnung im Magnetflussleiter 62
- 64: Leiterspule am Magnetflussleiter 62
- 68: Steuerung (mit elektrischer Leistungsquelle) für die Leiterspule 64

- 72: Bildsensor oder Kamera
- 73: Kameragehäuse
- 74: Schaltkreis zur Aufbereitung und/oder Verarbeitung des Bildsignals
- 76: Bauraum für Wärmequellen

- 82: Mantel
- 84: proximale Leitschaufel
- 86: distale Leitschaufel
- 88: Turbulator

## Patentansprüche

1. **Medizinisches Instrument** (10; 11), mit:
einer flüssigkeitsdichten **Hülle** (20);
einer **Wärmequelle** (22, 32, 72, 74);
einen **Oberflächenbereich** (40), der mit der Wärmequelle (22, 32, 72, 74) thermisch gekoppelt ist;
einem **Laufrad** (50) außerhalb der Hülle (20) zum Erzeugen eines Fluidstroms (57, 58, 59) an der Oberfläche (40);
einem **Magneten** (53) an dem Laufrad (50);
einer **Einrichtung** (60, 61, 62, 64, 68) zum Erzeugen eines veränderlichen Magnetfelds zum Bewegen des Laufrads (50), wobei die Einrichtung innerhalb der Hülle angeordnet ist,
**dadurch gekennzeichnet, dass** das medizinische Instrument ein **Endoskop** (11) mit einem langen dünnen Schaft (13) oder ein **Exoskop** (10) oder ein **Operationsmikroskop** ist, der Fluidstrom (57, 58, 59) ein Umgebungsluft-Strom ist und zur Abfuhr von durch die Wärmequelle erzeugter thermischer Leistung erzeugt wird.

2. Medizinisches Instrument (10; 11) nach dem vorangehenden Anspruch, bei dem die Einrichtung (60, 61, 62, 64, 68) zum Erzeugen eines veränderlichen Magnetfelds zum Erzeugen eines **rotierenden Magnetfelds** vorgesehen und ausgebildet ist.

3. Medizinisches Instrument (10; 11) nach einem der vorangehenden Ansprüche, bei dem die Einrichtung (60, 61, 62, 64, 68) zum Erzeugen eines veränderlichen Magnetfelds ferner zur **magnetischen Lagerung** des Laufrads (50) vorgesehen und ausgebildet ist.

4. Medizinisches Instrument (10; 11) nach einem der vorangehenden Ansprüche, bei dem das Laufrad (50) zum Erzeugen eines Fluidstroms in Richtung **von proximal nach distal** vorgesehen und ausgebildet ist.

5. Medizinisches Instrument (10; 11) nach einem der vorangehenden Ansprüche, wobei das medizinische Instrument (10; 11) ausgebildet ist, um einen **Fluidstrom** (57, 58, 59) **am distalen Ende** des medizinischen Instruments (10; 11) zu erzeugen.

6. Medizinisches Instrument (10; 11) nach einem der vorangehenden Ansprüche, bei dem das **Laufrad** (50) an oder nahe dem **distalen** Ende (14) des medizinischen Instruments (10, 11) angeordnet ist.

7. Medizinisches Instrument (10; 11) nach einem der vorangehenden Ansprüche, bei dem das **Laufrad** (50) in einer Richtung (51) parallel zu einer Längsachse (18) des medizinischen Instruments (10; 11) **verschiebbar** ist.

8. Medizinisches Instrument (10; 11) nach einem der vorangehenden Ansprüche, bei dem das Laufrad (50) von anderen Teilen (20) des medizinischen Instruments (10, 11) zerstörungsfrei und reversibel **getrennt** werden kann.

9. Medizinisches Instrument (10; 11) nach Anspruch 7 oder 8, bei dem zumindest entweder die Einrichtung (60, 61, 62, 64, 68) zum Erzeugen eines veränderlichen Magnetfelds zwischen mehreren Positionen verschiebbar ist oder **mehrere Einrichtungen** (60, 61, 62, 64, 68) zum Erzeugen eines veränderlichen Magnetfelds vorgesehen sind, um das Laufrad an mehreren Positionen antreiben zu können.

10. Medizinisches Instrument (10; 11) nach einem der vorangehenden Ansprüche, ferner mit:
einer **Steuerung** (68) zum Steuern der Einrichtung (60, 61, 62, 64) zum Erzeugen eines veränderlichen Magnetfelds,
wobei die Steuerung (68) ausgebildet ist, um zumindest entweder die Rotations**richtung** oder die Rotations**geschwindigkeit** des Laufrads (50) zu steuern.

11. Medizinisches Instrument (10; 11) nach dem vorangehenden Anspruch, ferner mit:
einer **Einrichtung** (36) zum Erfassen einer **Temperatur** der Wärmequelle (22, 32, 72, 74);
wobei die Steuerung (68) mit der Einrichtung (36) zum Erfassen der Temperatur gekoppelt und ausgebildet ist, um zumindest entweder die Rotationsrichtung oder die Rotationsgeschwindigkeit des Laufrads (50) **in Abhängigkeit von der Temperatur** der Wärmequelle (22, 32, 72, 74) zu steuern.

12. Medizinisches Instrument (10; 11) nach einem der vorangehenden Ansprüche, ferner mit:
einer **Leiteinrichtung** (82, 84, 86) zum Leiten oder Umlenken eines von dem Laufrad (50) erzeugten Fluidstroms (57).

13. Medizinisches Instrument (10; 11) nach einem der vorangehenden Ansprüche, bei dem zumindest entweder das Laufrad (50) oder die Leiteinrichtung (82, 84, 86) ausgebildet ist, um einen zumindest abschnittsweise **helikal** um einen Schaft (13) des medizinischen Instruments (10; 11) verlaufenden Fluidstrom (58) zu erzeugen.

14. Medizinisches Instrument (10, 11) nach einem der vorangehenden Ansprüche, ferner mit:
einer Einrichtung (88) zum Überführen einer **laminaren** Strömung (58) in eine **turbulente** Strömung (59).

## Claims

1. A medical instrument (10; 11) with:
a liquid-tight sheath (20);
a heat source (22, 32, 72, 74);
a surface area (40) thermally coupled to the heat source (22, 32, 72, 74);
a rotor (50) arranged outside the sheath (20) and serving to generate a flow of fluid (57, 58, 59) at the surface (40);
a magnet (53) at the rotor (50);
a means (60, 61, 62, 64, 68) for generating a variable magnetic field in order to move the rotor (50), the means being arranged inside the sheath,
**characterized in that** the medical instrument is an endoscope (11) with a long thin shaft (13) or an exoscope (10) or a surgical microscope, and **in that** the flow of fluid (57, 58, 59) is a flow of ambient air and is produced for removal of thermal output generated by the heat source.

2. The medical instrument (10; 11) as claimed in the preceding claim, in which the means (60, 61, 62, 64, 68) for generating a variable magnetic field is provided and designed to generate a rotating magnetic field.

3. The medical instrument (10; 11) as claimed in one of the preceding claims, in which the means (60, 61, 62, 64, 68) for generating a variable magnetic field is furthermore provided and designed for the magnetic support of the rotor (50).

4. The medical instrument (10; 11) as claimed in one of the preceding claims, in which the rotor (50) is provided and designed to generate a flow of fluid in a direction from proximal to distal.

5. The medical instrument (10; 11) as claimed in one of the preceding claims, wherein the medical instrument (10; 11) is designed to generate a flow of fluid (57, 58, 59) at the distal end of the medical instrument (10; 11).

6. The medical instrument (10; 11) as claimed in one of the preceding claims, in which the rotor (50) is arranged at or near the distal end (14) of the medical instrument (10, 11).

7. The medical instrument (10; 11) as claimed in one of the preceding claims, in which the rotor (50) is movable in a direction (51) parallel to a longitudinal axis (18) of the medical instrument (10; 11).

8. The medical instrument (10; 11) as claimed in one of the preceding claims, in which the rotor (50) can be separated from other parts (20) of the medical instrument (10, 11) in a non-destructive and reversible manner.

9. The medical instrument (10; 11) as claimed in claim 7 or 8, in which at least one of the means (60, 61, 62, 64, 68) for generating a variable magnetic field is movable between several positions and several devices (60, 61, 62, 64, 68) for generating a variable magnetic field are provided, so as to be able to drive the rotor at several positions.

10. The medical instrument (10; 11) as claimed in one of the preceding claims, the medical instrument further comprising:
a controller (68) for controlling the means (60, 61, 62, 64) for generating a variable magnetic field,
wherein the controller (68) is designed to control at least one of the rotational direction and the rotational speed of the rotor (50).

11. The medical instrument (10; 11) as claimed in the preceding claim, the medical instrument further comprising:
means (36) for detecting a temperature of the heat source (22, 32, 72, 74);
wherein the controller (68) is coupled to the means (36) for detecting the temperature and is designed to control at least one of the rotational direction and the rotational speed of the rotor (50) depending on the temperature of the heat source (22, 32, 72, 74).

12. The medical instrument (10; 11) as claimed in one of the preceding claims, further comprising:
a guiding means (82, 84, 86) for guiding or diverting a flow of fluid (57) generated by the rotor (50).

13. The medical instrument (10; 11) as claimed in one of the preceding claims, in which at least one of the rotor (50) and the guiding means (82, 84, 86) is designed to generate a flow of fluid (58) which extends, at least in part, helically around a shaft (13) of the medical instrument (10; 11).

14. The medical instrument (10, 11) as claimed in one of the preceding claims, the medical instrument further comprising:
means (88) for converting a laminar flow (58) to a turbulent flow (59).

## Revendications

1. Instrument médical (10 ; 11), comportant :
une enveloppe étanche aux liquides (20) ;
une source de chaleur (22, 32, 72, 74) ;
une zone de surface (40) thermiquement couplée à la source de chaleur (22, 32, 72, 74) ;
un rotor (50) disposé à l'extérieur de l'enveloppe (20) pour générer un écoulement de fluide (57, 58, 59) sur la surface (40) ;
un aimant (53) sur le rotor (50) ;
un dispositif (60, 61, 62, 64, 68) destiné à générer un champ magnétique variable, afin de déplacer le rotor (50), dans lequel le dispositif est disposé à l'intérieur de l'enveloppe,
**caractérisé en ce que** l'instrument médical est un endoscope (11) comportant une tige mince (13) ou un exoscope (10) ou un microscope chirurgical, **en ce que** l'écoulement de fluide (57, 58, 59) est un écoulement d'air ambiant et est généré pour évacuer la puissance thermique générée par la source de chaleur.

2. Instrument médical (10 ; 11) selon la revendication précédente, dans lequel le dispositif (60, 61, 62, 64, 68) destiné à générer un champ magnétique variable est prévu et conçu pour générer un champ magnétique tournant.

3. Instrument médical (10 ; 11) selon l'une quelconque des revendications précédentes, dans lequel le dispositif (60, 61, 62, 64, 68) destiné à générer un champ magnétique variable est en outre prévu et conçu pour suspendre magnétiquement le rotor (50).

4. Instrument médical (10 ; 11) selon l'une quelconque des revendications précédentes, dans lequel le rotor (50) est prévu et conçu pour générer un écoulement de fluide dans une direction allant des extrémités proximale à distale.

5. Instrument médical (10 ; 11) selon l'une quelconque des revendications précédentes, dans lequel l'instrument médical (10 ; 11) est conçu pour générer un écoulement de fluide (57, 58, 59) à l'extrémité distale de l'instrument médical (10 ; 11).

6. Instrument médical (10 ; 11) selon l'une quelconque des revendications précédentes, dans lequel le rotor (50) est disposé sur ou à proximité de l'extrémité distale (14) de l'instrument médical (10 ; 11).

7. Instrument médical (10 ; 11) selon l'une quelconque des revendications précédentes, dans lequel le rotor (50) peut être déplacé dans une direction (51) qui est parallèle à un axe longitudinal (18) de l'instrument médical (10 ; 11).

8. Instrument médical (10 ; 11) selon l'une quelconque des revendications précédentes, dans lequel le rotor (50) peut être séparé sans perturbation et de manière réversible d'autres parties (20) de l'instrument médical (10 ; 11).

9. Instrument médical (10 ; 11) selon la revendication 7 ou 8, dans lequel au moins soit le dispositif (60, 61, 62, 64, 68) destiné à générer un champ magnétique variable peut être déplacé entre plusieurs positions, soit plusieurs dispositifs (60, 61, 62, 64, 68) destinés à générer un champ magnétique variable sont prévus afin de pouvoir entraîner le rotor à plusieurs positions.

10. Instrument médical (10 ; 11) selon l'une quelconque des revendications précédentes, comportant en outre :
une système de commande (68) pour commander le dispositif (60, 61, 62, 64) destiné à générer un champ magnétique variable,
dans lequel le système de commande (68) est conçue pour commander au moins soit le sens de rotation, soit la vitesse de rotation du rotor (50).

11. Instrument médical (10 ; 11) selon la revendication précédente, comportant en outre :
un dispositif (36) destiné à détecter une température de la source de chaleur (22, 32, 72, 74) ;
dans lequel le système de commande (68) est couplée au dispositif (36) destiné à détecter la température et est conçue pour commander au moins soit le sens de rotation, soit la vitesse de rotation du rotor (50) en fonction de la température de la source de chaleur (22, 32, 72, 74).

12. Instrument médical (10 ; 11) selon l'une quelconque des revendications précédentes, comportant en outre :
un dispositif de guidage (82, 84, 86) destiné à guider ou dévier un écoulement de fluide (57) généré par le rotor (50).

13. Instrument médical (10 ; 11) selon l'une quelconque des revendications précédentes, dans lequel au moins soit le rotor (50), soit le dispositif de guidage (82, 84, 86) est conçu pour générer un écoulement de fluide (58) s'écoulant au moins partiellement de manière hélicoïdale autour d'une tige (13) de l'instrument médical (10 ; 11).

14. Instrument médical (10 ; 11) selon l'une quelconque des revendications précédentes, comportant en outre :
un dispositif (88) destiné à générer un courant laminaire (58) dans un courant turbulent (59).
